# EUROPÄISCHE PATENTSCHRIFT

(11) **EP 3 253 640 B1**
(45) Veröffentlichungstag und Bekanntmachung des Hinweises auf die Patenterteilung: **25.03.2020**
(21) Anmeldenummer: 16702048.6
(22) Anmeldetag: 22.01.2016
(51) Int. Cl.: B61L 5/10

(54) **WEICHENVERSTELLMECHANISMUS MIT EINEM ZWISCHEN DEN BEIDEN STOCKSCHIENEN ANGEORDNETEN VERSCHLUSS**
POINTS ADJUSTMENT MECHANISM HAVING A SWITCH LOCK BETWEEN THE TWO RAILS
DISPOSITIF D'AIGUILLAGE DOTÉ D'UN VERROU DISPOSÉ ENTRE LES DEUX CONTRE-AIGUILLES

(30) Priorität: 02.02.2015 EP 15153488
(43) Veröffentlichungstag der Anmeldung: 13.12.2017
(73) Patentinhaber: Siemens Mobility AG, 8304 Wallisellen (CH)
(72) Erfinder: ACKERET, Walter, 8472 Seuzach (CH); BIERI, Natalie, 8037 Zürich (CH); FELIX, Jon, 8152 Opfikon (CH)
(74) Vertreter: Deffner, Rolf
(86) Internationale Anmeldenummer: PCT/EP2016/051352
(87) Internationale Veröffentlichungsnummer: WO 2016/124425

(56) Entgegenhaltungen:
- EP-A1- 1 110 841
- EP-A1- 1 378 417
- DE-A1- 3 543 403

## Beschreibung

Die vorliegende Erfindung betrifft einen Weichenverstellmechanismus mit einem zwischen den beiden Stockschienen angeordneten Verschluss.

Zur Umstellung von Weichen ist eine Vielzahl unterschiedlicher Umstellvorrichtungen bekannt. Häufig werden Klammerspitzen- oder Klinkenverschlüsse eingesetzt. Letztgenannter Klinkenverschluss ist aus der europäischen Patentanmeldung EP 0 624 508 A1 und in ähnlicher Konfiguration auch aus der deutschen Anmeldung DE 35 43 403 A1 bekannt.

Die Klinke eines Klinkenverschlusses ist verschwenkbar an einem Verschlusslager gehalten. An dem Verschlusslager wird weiter direkt oder über ein Zwischenbauteil die Weichenzunge befestigt, zu deren Verstellung und Verriegelung der Klinkenverschluss eingesetzt wird. Der Klinkenverschluss hat somit die Aufgabe, die an einer Stockschiene anliegende Weichenzunge sowie die abliegende Weichenzunge in einer geometrisch exakt definierten Position festzuhalten und mechanisch zu verriegeln. Die Art der mechanischen Verriegelung erfolgt mit Vorteil formschlüssig. Aufgrund der Sicherheitsrelevanz der beschriebenen Funktionalität bestehen an die Herstellung und die Wartung von Weichen erhöhte Ansprüche, sodass es im Besonderen eine Aufgabe der Konstruktion ist, den Weichenverschluss möglichst einfach und sicher aufzubauen. Ebenso liegt derselbe Massstab an der Fertigung an, die die erforderlichen Teile des Klinkenverschlusses fehlerfrei bereitzustellen hat.

Die Verschlussklinke - oder einfach Klinke genannt - wird in dem Verschlusslager mittels eines Exzenterbolzens schwenkbar gehalten. Mittels des Exzenterbolzens kann die Position der Verschlussklinke in einer Richtung, die senkrecht zur Ausdehnung der Stockschiene verläuft, verschoben werden. Diese Verschiebung soll das korrekte Anliegen der Weichenzunge an der Stockschiene herbeiführen. Nachteilig an der Exzenterlösung ist es jedoch, dass der Einstellbereich relativ klein ist und dass sich der relative Abstand der am Fusse des Verschlusslagers vorgesehenen Verdrehsicherung zum an der Stockschiene montierten Verschlussträger ändert. Zudem wird durch die Verstellung der Position der Verschlussklinke mit dem Exzenterbolzen auch der Drehpunkt der Klinkenlagerung entsprechend mitverschoben. Im Feld ist es daher die Regel so, dass jede Weiche mittels der Bevorratung verschiedener Verschlusslager und Verschlussklinken im Rahmen der jeweiligen Verstellbarkeit individuell anzupassen sind, was nicht nur einen hohen Aufwand der Bewirtschaftung dieser Vielzahl von Bauteilen bedingt, sondern eben auch in der Konstruktion und der Fertigung immer wieder zu vergleichsweise kostspieligen Kleinstserien führt.

Die bekannte Bauart eines Aussenverschlusses (beispielsweise der Klinkenverschluss) verriegelt die Weichenzunge formschlüssig mittels einer beweglichen Verschlussklinke. Solche Verschlussklinken werden mittels einer geeigneten Lagerung an beide Weichenzungen befestigt und verriegeln damit die Weichenzunge direkt mit der Stockschiene. Eine solche Anordnung gewährleistet in Kombination mit einem Weichenantrieb eine sichere Durchfahrt eines Schienenfahrzeuges. Aufgrund der unterschiedlichen Spurweiten (Meterspur, Kapspur, Normalspur, Breitspur etc.) und in Kombination mit der hohen Varianz der Profile für Weichenzungen bzw. Stockschienen entsteht ein breites Spektrum an geometrischen Anforderungen für die Realisierung der erwähnten Aussenverschlüsse. In der Regel wird für jede Ausführungsvariante ein eigener Verschlusstyp benötigt, was aus Sicht der Konstruktion bzw. Fertigung der Verschlüsse suboptimal ist.

Heute kommen entweder Aussen- oder Innenverschlüsse zum Einsatz. Welches Prinzip konkret eingesetzt wird, ist von verschiedenen Faktoren abhängig. Einerseits geht es um die Geschwindigkeit der Züge, andererseits um die Bauart der Weichenzungen. Weitere Aspekte wie beispielsweise die vorherrschenden Umweltbedingungen spielen ebenfalls eine Rolle. Konventionelle (Aussen)Verschlüsse haben folgende Nachteile:
a) Der Verschluss muss für jedes Schienenprofil und spezifisch für jede Spurweite neu konstruiert werden. Daraus resultiert eine hohe Anzahl unterschiedlicher Ausführungen.
b) Aufgrund der Vielfalt der Verschlüsse sind kaum Skaleneffekte im Rahmen der Fertigung realisierbar.
c) Klimatische bzw. Umwelteinflüsse können den Einsatz konventioneller Aussenverschlüsse verunmöglichen.

Der vorliegenden Erfindung liegt daher die Aufgabe zugrunde, einen einfach aufgebauten und funktionssicheren

Klinkenverschluss anzugeben, bei dem die Bauart einfacher an die verschiedenen Spurweiten und Profile der Weichenzungen anpassbar ist ohne die vorteilhaften Verschlusswirkungen bekannter Weichenverstellmechanismen aufgeben zu müssen.

Diese Aufgabe wird erfindungsgemäss durch einen Weichenverstellmechanismus gemäß Anspruch 1 gelöst.

Auf diese Weise wird ein zentral zwischen den Stockschienen angeordneter Verschlusskern für den Weichenmechanismus geschaffen, wobei die bewährte Funktionalität der Verschlussklinke beibehalten wird. Das Konzept des universellen Verschlusskernes reduziert den Entwicklungsaufwand insbesondere im Hinblick auf neue Produkt/Marktkombinationen erheblich. Die Anpassungsaufwände für allfällige Markteintritte werden deutlich reduziert bei gleichzeitiger Volumenausweitung, weil für unterschiedliche Weichen dasselbe Konzept verwendet werden kann. Insgesamt resultieren daher die folgenden Vorteile:
a) Die Eintrittskosten für neue Märkte werden reduziert.
b) Die time-to-market für neue Märkte wird deutlich verringert.
c) Die Produktvielfalt im Bereich der Weichenstellsysteme wird reduziert.
d) Aufgrund der Möglichkeit zur Vormontage wird der Aufwand für die Montage vor Ort deutlich vereinfacht.

Der erfinderische Wert liegt daher in der Realisierung einer universell verwendbaren Verschlusseinheit, welche durch einfache Adapterteile bzw. aufgrund unterschiedlich langer Zungenstellstangen an verschiedene Zungenprofile bzw. Weichentypen angepasst werden kann.

In einer weiteren vorteilhaften Ausgestaltung der vorliegenden Erfindung kann das Verschlusslager auf einer Führungsschiene senkrecht zur Richtung der Stockschiene verschiebbar gelagert sein. Diese Führungsschiene ist dabei in einfacher Weise mit der Beschlussbefestigung verbindbar und gibt dem Laufweg des Verschlusslagers die notwendige statische Unterstützung.

Auch bei diesen vorstehend genannten Ausführungsformen der vorliegenden Erfindung ist eine Trennung der elektrischen Potentiale der beiden Stockschienen und den ihnen zugeordneten Weichenzungen seitenweise zu erzielen. Hierzu kann eine einfache Lösung erzielt werden, wenn zwischen dem Adapterstück und der Weichenzunge oder zwischen dem Verschlusslager und der Zungenstellstange eine elektrische Isolierung angeordnet ist.

Weiter kann es zur Erhöhung der Flexibilität des Weichenverstellmechanismus vorgesehen sein, dass das Verschlusslager zur Befestigung der Zungenstellstange einen Excenter-Bolzen aufweist. Auf diese Weise können kleinere Toleranzen zwischen dem Verschlusslager und der Weichenzunge durch das entsprechende Verdrehen des Excenter-Bolzens ausgeglichen werden.

Weitere vorteilhafte Ausgestaltungen sind in den übrigen Unteransprüchen ausgeführt.

Vorteilhafte Ausgestaltungen der vorliegenden Erfindung werden anhand einer Zeichnung näher erläutert. Dabei zeigen:
- Figur 1: in schematischer Darstellung eine seitliche Ansicht eines Klinkenverschlusses mit Verschlussträger und Verschlusslager gemäss dem Stand der Technik;
- Figur 2: in schematischer Darstellung einen Klinkenverschlusses mit zentral angeordnetem Verschlusslager im Rohzustand; und
- Figur 3: in schematischer Darstellung einen Klinkenverschlusses mit zentral angeordnetem Verschlusslager im eingebauten Zustand.

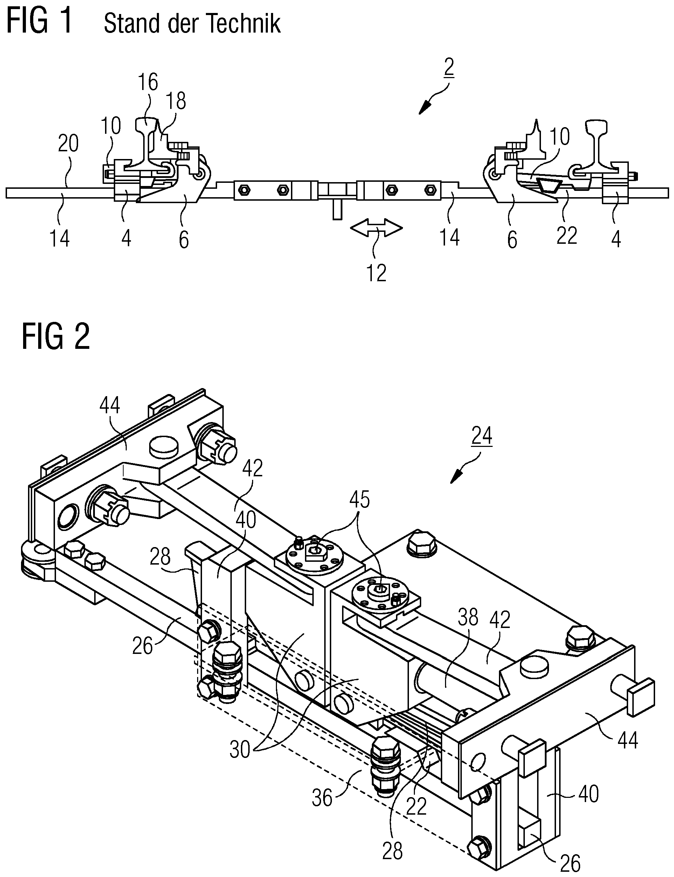

Die Figur 1 zeigt in schematischer Darstellung eine seitliche Ansicht eines Klinkenverschlusses 2 gemäss dem Stand der Technik mit beidseitig angeordneten Verschlussträgern 4 und einem Verschlusslager 6. In jedem Verschlusslager 6 ist eine Verschlussklinke 10 in einer Richtung senkrecht zu einer Verstellrichtung 12 verschwenkbar gehalten. Der Klinkenverschluss 2 wird mittels einer Schieberstange 14 (oder auch Nockenstange genannt) in die eine oder andere Richtung bewegt. Bezüglich der Ausgestaltung eines Klinkenverschlusses wird auf die europäische Patentanmeldung EP 0 624 508 A1 verwiesen, die hiermit als komplett eingeführt gilt. In der gezeigten Darstellung ist auf der linken Seite eine an einer Stockschiene 16 anliegende Weichenzunge 18 ersichtlich, während entsprechend auf der rechten Seite die Weichenzunge von der Stockschiene abliegt. Auf der linken Seite ist die Verschlussklinke 10 durch die entsprechende Stellung der Nockenstange 14 im Verschlussträger 4 und hinter der äusseren Kante der Stockschiene 16 verriegelt. Beim Lösen dieser Verriegelung fährt die Nockenstange 14 in der zeichnerischen Darstellung in die rechte Richtung und erlaubt der Verschlussklinke 10 in einem Bereich 20 der Nockenstange 14 aus der verriegelten Stellung nach unten auf die Nockenstange 14 zu fallen. Die Verschlussklinke 10 kann so unter der Stockschiene 16 hindurch bewegt werden. Der genau umgekehrte Vorgang passiert dann auf der anderen Seite, wo die Verschlussklinke 10 zum Ende der Verstellbewegung auf eine an der Nockenstange 14 angeformte Nocken 22 aufläuft und somit unter der anderen Stockschiene verriegelt wird.

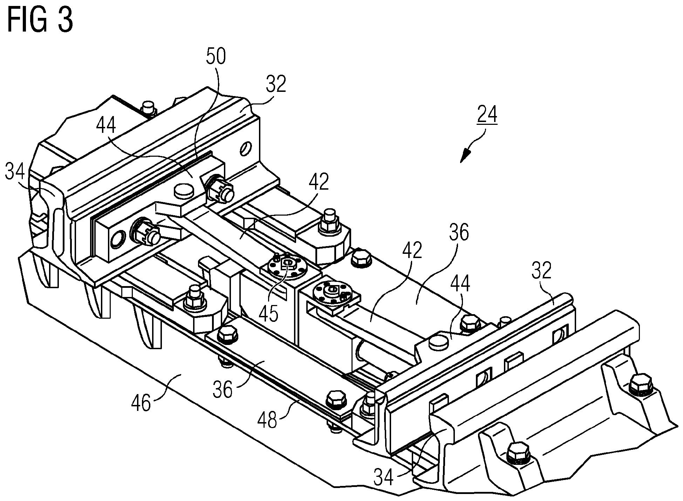

Bei dem in Figur 2 gezeigten erfindungsgemässen Klinkenverschluss 24 werden die vorstehend beschriebenen Verriegelungsmechanismen und im Besonderen das Zusammenwirken einer Nockenstange 26 mit einer Verschlussklinke 28 beibehalten. Gegenüber der Darstellung gemäss der Figur 1 sind nun aber die folgenden Bauteile modifiziert: Je ein Verschlusslager 30 ist nun in einer zentralen zwischen Weichenzungen 32 und Stockschiene 34 (vgl. Figur 3 im Einbauzustand) angeordneten Verschlussbefestigung 36 (in Figur 2 gestrichelt dargestellt) in Verstellrichtung der Nockenstange 26 verschiebbar gelagert. Hierzu ist eine ebenfalls mit der Verschlussbefestigung 36 verbundene Führungsstange 38 vorgesehen. In der zeichnerischen Darstellung befindet sich jeweils ein Verschlussträger 40 rechts und links von den Verschlusslagern 30. Auf diesen Verschlussträgern 40, die ebenfalls mit der Verschlussbefestigung 36 verbunden sind, liegt die Nockenstange 26 in ihrer Verstellrichtung senkrecht zu den Stockschienen 34 verschiebbar auf.

An den Verschlusslagern 30 koppeln Zungenstellstangen 42 an, die mittels Exzenter-Bolzen 45 mit den Verschlusslagern 30 verbunden sind. Die Zungenstellstangen 42 greifen mit ihren gegenüberliegenden Enden an Adapterteilen 44 an, die zur Anbindung der Weichenzungen 32 an den Klinkenverschluss 24 verwendet werden. Dabei lassen sich gerade mit der individuellen Anpassbarkeit der Adapterteile 44 die besonderen Vorteile des zentralen Verschlusskerns (Verschlussbefestigung 36) erzielen, weil einzigallein an dieser Stelle eine Verbindung zu den Weichenzungen 32 zu erzielen ist, ohne dass das Verschlusslager 30 selbst an der Weichenzunge 32 oder der Verschlussträger 40 an der Stockschiene 34 ankoppeln müssten wie dies jedoch in nachteiliger Weise im Stand der Technik der Fall ist. Zudem kann an den Adapterteilen 44 auch die galvanische Trennung der beiden Weichenzungen 32 vorgenommen werden, zum Beispiel durch die hier nur sehr rudimentär gezeigten Isolierlagen 50.

Figur 3 zeigt nun den Klinkenverschluss 24 im Einbauzustand; und zwar vorliegend in eine Hohlschwelle 46 eingebaut. Die Verschlussbefestigung 36 ist nun auf Flanschen 48 der aufsteigenden Kanten der im Profil U-förmigen Hohlschwelle 46 eingebaut. Feinjustierungen des Abstandes der Verschlusslager 30 zu den Weichenzungen 32 können mit den Exzenter-Bolzen 45 vorgenommen werden. Alternativ zu dem hier gezeigten Einbau in einer Hohlschwelle 46 oder auch Kastenschwelle genannt (hier ersetzt die Hohlschwelle einen normalen Schwellenkörper) kann dieser Klinkenverschluss 24 auch in einem sogenannten Schwellenfach zwischen benachbarten Schwellenkörpern eingebaut werden.

## Patentansprüche

1. Weichenverstellmechanismus (24) für zwei zwischen zwei gegenüberliegenden Stockschienen (34) angeordneten beweglichen Weichenzungen (32) mit einem ebenfalls zwischen den gegenüberliegenden Stockschienen (34) angeordneten Verschlusslager (30), wobei:
a) das Verschlusslager (30) von einer **zwischen den Stockschienen angeordneten** Verschlussbefestigung (36) getragen wird;
b) die Verschlussbefestigung (36) auf zwei benachbart angeordneten Schwellenkörpern oder auf beiden Flanschen einer Hohlschwelle (46) befestigbar ist;
c) eine Verriegelung des Weichenstellmechanismus durch das Zusammenwirken von einer in einem **zentralen** Verschlussträger (40) geführten Nockenstange (26) und mindestens einer an dem Verschlusslager (30) schwenkbar gelagerten Verschlussklinke (28) erzielt wird, wobei die Schwenkachse waagrecht ausgerichtet ist;
d) der Verschlussträger (40) mit der Verschlussbefestigung (36) verbunden ist;
e) ausgehend von dem Verschlusslager (30) mittels einer daran befestigten Zungenstellstange (42) und einem daran befestigten Adapterstück (44) ein Angriff an der Weichenzunge (32) vorgesehen ist, und
f) die Nockenstange (26) von einem Weichenantrieb zwischen den beiden Endpositionen der Weichenzungen (32) bewegbar ist.

2. Weichenverstellmechanismus nach Anspruch 1,
**dadurch gekennzeichnet, dass**
das Verschlusslager (30) auf einer Führungsschiene (38) senkrecht zur Richtung der Stockschiene (34) verschiebbar gelagert ist.

3. Weichenverstellmechanismus nach Anspruch 1 oder 2,
**dadurch gekennzeichnet, dass**
zwischen dem Adapterstück (44) und der Weichenzungen (32) oder zwischen dem Verschlusslager (30) und der Zungenstellstange (42) eine elektrische Isolierung (50) angeordnet ist.

4. Weichenverstellmechanismus nach einem der Ansprüche 1 bis 3,
**dadurch gekennzeichnet, dass**
das Verschlusslager (30) zur Befestigung der Zungenstellstange (42) einen Exzenter-Bolzen (45) aufweist.

## Claims

1. Points adjustment mechanism (24) for two movable point tongues (32) arranged between two opposing stock rails (34) with a lock housing (30) likewise arranged between the opposing stock rails (34), wherein:
a) the lock housing (30) is supported by a lock fastening (36) **arranged between the stock rails;**
b) the lock fastening (36) can be fastened to two adjacently arranged sleeper bodies or to both flanges of a hollow sleeper (46) ;
c) a locking of the points adjustment mechanism by the interaction of a cam rod (26) guided in a **central** lock support (40) and at least one locking pawl (28) pivotably mounted on the lock housing (30) is achieved, wherein the pivot axis is aligned horizontally;
d) the lock support (40) is connected to the lock fastening (36) ;
e) starting from the lock housing (30) by means of a tongue adjusting rod (42) fastened to it and an adapter piece (44) fastened to it, a point of attachment to the point tongue (32) is provided, and
f) the cam rod (26) can be moved by a point machine between the two end positions of the point tongues (32).

2. Points adjustment mechanism according to claim 1,
**characterised in that**
the lock housing (30) is displaceably mounted on a guide rail (38) perpendicular to the direction of the stock rail (34).

3. Points adjustment mechanism according to claim 1 or 2,
**characterised in that**
electrical insulation (50) is arranged between the adapter piece (44) and the point tongues (32) or between the lock housing (30) and the tongue adjusting rod (42).

4. Points adjustment mechanism according to one of claims 1 to 3, **characterised in that**
the lock housing (30) has an eccentric bolt (45) for fastening the tongue adjusting rod (42).

## Revendications

1. Mécanisme de déplacement d'aiguille (24) destiné à deux lames d'aiguille (32) mobiles disposées entre deux contre-aiguilles opposées (34), et comportant un palier de fermeture (30) également disposé entre les contre-aiguilles opposées (34), dans lequel :
a) le palier de fermeture (30) est supporté par une fixation de fermeture (36) disposée entre les contre-aiguilles ;
b) la fixation de fermeture (36) peut être fixée sur deux corps de traverse adjacents ou sur les deux brides d'une traverse creuse (46) ;
c) un verrouillage du mécanisme de déplacement d'aiguille est obtenu par coopération d'une barre à cames (26) guidée dans un support de fermeture central (40) et d'au moins un cliquet de fermeture (28) monté à pivotement sur le palier de fermeture (30), l'axe de pivotement étant orienté à l'horizontale ;
d) le support de fermeture (40) est relié à la fixation de fermeture (36) ;
e) un engagement avec la lame d'aiguille (32) est prévu à partir du palier de fermeture (30), au moyen d'une barre d'actionnement d'aiguille (42) fixée audit palier et d'une pièce d'adaptation (44) fixée à ladite barre, et
f) la barre à cames (26) peut être déplacée par un entraînement d'aiguille entre les deux positions extrêmes des lames d'aiguille (32).

2. Mécanisme de déplacement d'aiguille selon la revendication 1,
**caractérisé en ce que** le palier de fermeture (30) est monté de manière mobile perpendiculairement à la direction de la contre-aiguille (34) sur une coulisse de guidage (38).

3. Mécanisme de déplacement d'aiguille selon la revendication 1 ou 2,
**caractérisé en ce qu'**une isolation électrique (50) est disposée entre la pièce d'adaptation (44) et les lames d'aiguille (32) ou entre le palier de fermeture (30) et la barre d'actionnement d'aiguille (42).

4. Mécanisme de déplacement d'aiguille selon l'une des revendications 1 à 3,
**caractérisé en ce que** le palier de fermeture (30) est pourvu d'un boulon d'excentrique (45) pour la fixation de la barre d'actionnement d'aiguille (42).
